# EUROPEAN PATENT APPLICATION

(11) **EP 4 725 849 A2**
(43) Date of publication of application: **15.04.2026**
(21) Application number: 25227392.5
(22) Date of filing: 25.10.2023
(51) Int. Cl.: B64U 10/13

(54) **SYSTEM FOR TESTING SOIL PROPERTIES AND METHOD FOR CONDUCTING SOIL PROPERTY TESTS USING THIS SYSTEM**

(30) Priority: 26.10.2022 PL 44264922
(62) Divisional of application: 23800970.8
(71) Applicant: NIRBY SPOLKA Z OGRANICZONA ODPOWIEDZIALNOSCIA, 36-002 Jasionka (PL)
(72) Inventor: LAZAREK, Piotr, Paw owice (PL)
(74) Representative: JWP Patent & Trademark Attorneys

(57) **Abstract**

A soil properties in-ground probe 1 having a tubular body 101, containing a head 106, in the side of which is an inspection hole 107 protected by glass, the head 106 containing elements of a system for carrying out a measurement of soil properties, **characterized in that** at least one projection, preferably in the form of a blade 105, is located on the outer surface of the body 101 of the probe 1, with at least part of its essential surface contained within a helical surface, preferably rectilinear, determined around the longitudinal axis of the probe 1.

Unmanned system 2 for probe 1 **characterized in that** the probe 1 mounted on the body of the system 2 is positioned perpendicularly to the plane of the soil to be tested.

The method for carrying out a soil property test comprising inserting a probe 1 into the soil, **characterized in that** the probe 1 is delivered by an unmanned system 2 to the measurement site, inserted by a reciprocating motion into the soil, after which the direction of the reciprocating motion is changed and the probe 1 is brought out of the soil, whereby at least one soil property test is carried out when the probe 1 is embedded in the soil, whereby the test is carried out before the probe 1 is brought out of the ground spectroscopically and preferably with non-spectroscopic sensors of the head 106 of the probe 1, whereby during the implementation of the method the probe 1 is embedded in the reciprocating drive mechanism 207 of the unmanned system 2 and the sequences of insertion of the probe 1 into the ground, the measurement sequences and the sequences of bringing the probe 1 out of the ground are repeated cyclically at all predetermined points of ground testing in a given area.

## Description

The object of the invention is a system for testing soil properties comprising unmanned aerial vehicle coupled with an in-ground probe, wherein a probe contains instrumentation for performing spectroscopic test of soil properties and which may contain additional sensors for performing non-spectroscopic test of soil properties. In addition, the object of the invention is a method for conducting a soil property test with a soil testing system comprising unmanned aerial vehicle coupled with an in-ground probe according to the invention, wherein unmanned aerial vehicle of the system is adapted for carrying and inserting the probe with which it is coupled, into the ground to be tested, as well as for implementing a method for conducting soil property tests.

There are in-ground probes, unmanned platforms (systems) for transporting in-ground probes and inserting them into the investigated soil known from practice and the patent literature, and there are known methods to carry out investigations of soil properties based on spectroscopy or measurement sensors using measurements of electrical conductivity, temperature or soil acidity for measuring the characteristics of the investigated soil.

From patent application US2021356622, there is known an in-ground portable optical probe for spectroscopic investigation of soil properties. The disclosed probe has a tubular body conventionally terminated at the bottom by a conical spike, wherein the tubular body of the probe near the spike contains elements of a system for spectroscopic measurement such as light sources, elements changing the direction of the light beam, inspection holes in the shell of the tubular body for illuminating adjacent soil in a situation where the probe is driven into the soil to the side of the body, with visible, infrared or ultraviolet light. The disclosed probe further includes a spectrometer for analyzing the spectrum reflected from the illuminated ground. In addition, it is disclosed that the upper body of the probe contains elements (screen, buttons) for controlling the light system and displaying the measurement results to the user, and, depending on the internal configuration of the elements of the optical system, may also contain elements constituting a light tract. In addition, it is disclosed that the probe body may include a sensor provided with electrodes for measuring soil electroconductivity, and that the probe may communicate wirelessly with an external computer for transmission of output and input signal(s) (control of probe systems, transmission of measurement results).

The document also discloses how to conduct a soil property test with it, which involves the user driving the probe into the ground, exposing a layer of soil adjacent to the side of the probe body through an inspection hole, collecting the reflected spectrum and transmitting it for spectroscopic analysis. After the measurement, the probe is pulled out of the ground. In addition, it is indicated that it is advantageous to make an omnidirectional measurement with the probe due to the use of an inspection hole in the probe design, which is an annular section of the probe shell made of a transparent material (quartz glass, sapphire glass).

Patent application US2021223226, on the other hand, discloses a device for measuring soil properties such as electrical conductivity, pH, temperature, absorption spectrum (NIR to UV), having a tubular body conventionally terminated at the bottom with a measuring head provided with parallel spikes (probes) connected to sensors for measuring soil properties. It is also provided that the probe head may, between the spikes, contain a spectrometer, Preferably offset into the head and connected to the inspection hole by an optical system. It is further disclosed that the spectrometer head may be pivotally mounted on the probe body, and that the probe head may include a movable aperture for self-calibration of the spectrometer.

In addition, the document discloses a method of conducting a probe test, including: driving the probe's picks into the ground and pressing the spectroscope's inspection hole to its surface, taking up to two or Preferably three ground parameters, combining the measurement data in customized software to determine the measurement boundary conditions on the tested acreage, with the measurement data being sent to a central unit or decentralized computer network for calculations and processing of the measurement results.

The document also reveals that the probe and an external computer can be built on a transport device suitable for offline measurements.

Patent application US2017370064 discloses a VIS-NIR penetrometer for testing soil properties by reflectance spectroscopy. The disclosed penetrometer has a tubular body conventionally terminated at the bottom by a conical spike, with a head formed in the body capable of containing several NIR or VIS spectroscopic sensors, capacitive sensors, displacement sensors, moisture sensors. The body at the opposite end is further provided with a telescopic link. In addition, the head of the disclosed penetrometer may include optical elements (mirrors, lenses) for changing the direction and focusing of the light beam. According to the disclosure, the optical module elements may be connected to more than one detector built inside the penetrometer. The use of optical fibres to connect the detector to the optical system of the probe is also provided. It is further disclosed that the penetrometer can be attached to equipment such as trucks, ATVs, hydraulic soil sampling systems attached to agricultural tractors. In addition, according to the disclosure, measurement with the probe is carried out by illuminating a layer of soil adjacent to the side of the probe body and collecting the reflected spectrum when the probe is driven into the soil, and provision is made for continuous measurement during slow penetration of the probe into the soil.

From patent application US2011106451, a multi-sensor system for testing soil properties and a method for mapping soil in three dimensions is known. The document discloses that the system essentially contains two components: a multi-sensor plate dragged along the soil surface to measure soil parameters in the horizontal axis, and a hydraulically operated tube probe to take measurements deep into the soil. A set of spectrometers (NIR and VIS) and optical sensors, soil conductivity, the force required to drive the probe into the soil, and a soil temperature sensor are envisioned. The method of measurement according to the disclosure includes the first pass of a set connected to a wheeled cart coupled to an agricultural tractor, for determining in the horizontal plane the place of insertion of the soil probe. The control software then determines the insertion location of the in-ground probe based on the georeferenced surface measurement. After measuring with the doghole probe, it is possible to replace it with a soil core sampling probe. In doing so, the plate module and the topsoil probe module are mounted on a wheeled cart pulled by a well-known agricultural tractor, with the cart additionally containing a computer with implemented software for data analysis and georeferenced mapping.

Patent application US2018364157, on the other hand, discloses an unmanned apparatus for conducting a survey of the condition of land (or crops) by means of image analysis and spectral analysis of samples. Preferably, the drone is an optocopter or a riding drone on whose extended arm a multispectral camera is mounted. In addition, the drone includes a second measurement system for ground spectroscopic analysis. In addition, it is disclosed that the drone has a probing system for soil samples, with the probing system being seated below the plane determined by the drone's skids for enabling sampling without landing. The collected sample is subjected to spectroscopic analysis by a second system, with the probing system having the ability to store anomalous samples and the drone having the ability to assign a measurement value to a specific retained soil sample. According to the disclosure, soil testing and georeferencing with a drone according to the disclosure involves making two flights (passes). In the first, the drone performs image analysis from the multispectral camera for indicating, prior to the second flight (pass) by the user, the location of soil sampling with the probing system for spectroscopic analysis of a particular sample. The drone then makes a second flight (pass) to the locations indicated by the user and takes a soil sample from the indicated location, followed by its spectroscopic measurement. If a large deviation in the spectroscopy result is detected, the drone can secure a soil sample for further investigation and assign a measurement value to it. In addition, based on the data from two flights (passes), the disclosed unmanned system creates a georeferenced map to determine the condition of the cultivation area or assign GPS data to a specific sample measurement to enable targeted cultivation treatments.

In addition, it was disclosed that the drone can communicate wirelessly with an external user-operated computer or can be equipped with circuitry and software that allows it to operate in autonomous mode without radio transmission of measurement data to an external receiver.

Solutions known from the state of the art have a number of disadvantages. The in-ground probes that allow measurements at different depths of the ground and omnidirectional measurements require direct handling by the user, including driving them into the ground and moving them between sites for soil properties testing. On the other hand, systems adapted for transfer without direct user involvement are systems of large size and weight - necessitating, among other things, that they be pulled by agricultural tractors and making it impossible to implement known solutions on unmanned flying units. Revealed in the state of the art, survey systems operating on the basis of unmanned platforms, including flying ones, do not allow a spectroscopic survey of the ground essentially below ground level. Moreover, the known probes do not have solutions to facilitate their insertion and removal from the ground. In addition, the state of the art does not disclose a method of measurement that would allow calibration, calibration and refinement of spectroscopic measurements with additional sensors operating according to a technology other than spectroscopy, among others. Nor has the state of the art disclosed the possibility of using multiple unmanned systems to optimize the soil survey process in one or more agricultural fields.

The aim of the invention is to eliminate the disadvantages known from the state of the art by developing a system for investigating the properties of soil, comprising unmanned aerial vehicle coupled with an in-ground probe, equipped with components enabling spectroscopic measurements as well as measurements using other methods for investigating the properties of soil, and also having technical means for facilitating the insertion and removal of the probe from the soil and, moreover, enabling measurements to be carried out without the need for the user to be directly involved in driving the probe in and out of the soil, and allowing the probe to be inserted into the soil using a relatively lightweight aerial vehicle without the risk of causing the probe carrier to topple over. Furthermore, the aim of the invention is to develop such a design of an in-ground probe that would allow to test of soil properties along a helical trajectory for the widest possible coverage of the ground surface adjacent to the side of the probe carrier as it is plunged into and withdrawn from the ground. In addition, the purpose of the invention is to develop an unmanned aerial vehicle suitable for transporting the probe along with a method of delivering the unmanned aerial vehicle(s) to the site where the soil properties survey is performed, and to develop a method of conducting a soil properties survey with a soil testing system comprising unmanned aerial vehicle coupled with the probe, with the ability to create georeferenced maps of the survey results for targeted tillage operations.

Unexpectedly for specialists, the technical issues posed against the prior-art have been achieved by developing a soil testing system comprising unmanned aerial vehicle coupled with an in-ground probe with a tubular body provided with technical means for screwing it in and out of the ground, with the system being adapted to realize the method of carrying out a study of the properties of the soil using a probe of the disclosed design.

A coupled with an unmanned aerial vehicle in-ground probe according to the invention has a tubular body having a measuring part, the first, conventionally lower, conical end of which contains a head, in the side of which a glass-protected inspection hole is formed. In addition, the head contains inside a measuring instrumentation for carrying out a test of soil properties, whereby at least one projection, Preferably in the form of a blade, is located on the outer surface of the probe body, at least within the head, and Preferably within the head and the tubular part of the probe body, with at least part of its essential surface contained within a helical surface, Preferably rectilinear, defined around the longitudinal axis of the probe. As is obvious to the specialist, a helical surface means any surface formed by any curve by its helical motion about the longitudinal axis of the probe. What is also obvious to the specialists, under the term - curve should be understood also a special favorable case of it, i.e. a straight line.

According to the invention, the head of probe coupled with an unmanned aerial vehicle includes, depending on the target configuration, at least one of the following: a spectrometer for studying the reflectance spectrum of the soil under study, technical means for transmitting or changing the direction or focus of the light beam (for example: mirrors, lenses, optical fibres), a light source - emitting radiation in a range selected for the range of operation of the spectrometer, a sensor for studying the properties of the soil non-spectroscopically, for example, non-restrictively: an electrical conductivity sensor, a moisture sensor, a temperature sensor or a soil acidity sensor.

Preferably, inside the body of the probe there is located at least one conductor (electrical, optical fiber) running from the probe head towards the other end of the tubular body, constituting the sedimentary part thereof, opposite to the first end thereof, for control or powering of the measuring instrumentation located in the probe head or for transmission of light radiation, whereby both transmission of light radiation from a light source located outside the probe head according to the invention and transmission of the reflected spectrum to a spectrometer located outside the probe head are permitted. In this case, the optical fiber is a bundle of optical fibres, with at least one optical fiber strand of the bundle functionally coupled to an external spectrometer and at least one optical fiber strand, independent of the bundle coupled to the spectrometer, functionally coupled to an external light source.

Preferably, at least one conductor (electrical, fiber optic) running inside the probe body is rotatably arranged inside the probe body, whereby, depending on the probe head configuration and the number and type of bearing conductors used in a given probe configuration and the necessity of positioning the conductors within the inner space of the body resulting from the spatial positioning of the various elements of the measuring instrumentation, according to the invention, at least one bearing point is provided, preferably being a ball bearing or a plain bearing, through the inner ring of which the conductor(s) pass, within the inner space of the body (or head) of the probe according to the invention. Preferably, the bearing points are established in baffles, preferably dividing the inner space of the probe body transversely to its longitudinal axis, whereby, preferably, the axes of the bearing points are angled with respect to the longitudinal axis of the probe body in case it is necessary to ensure the proper passage and alignment of the conductors in a given probe configuration.

The use of bearing points allows the probe body to rotate freely as it is screwed into the ground or as it is twisted out of the ground around the wires running inside the probe body, eliminating the risk of twisting or otherwise damaging the wires connecting the probe head to the other end of the probe and beyond, with an unmanned aerial vehicle of the system for operating the probe according to the invention.

Preferably, an example of realization of the probe according to the invention is allowed, in which within the body of the probe are embedded, in addition to the measuring instrumentation of the head, other components responsible for acquisition, processing, or wireless transmission of measurement data - powered from a battery built within the body of the probe. In such a configuration, the probe according to the invention is not wired to the unmanned aerial vehicle of the system according to the invention, but contains within its body elements for wireless communication with the control unit of the unmanned aerial vehicle of the system or with an external receiver, located outside the perimeter of the probe and the unmanned aerial vehicle of the system.

Furthermore, preferably the probe includes known technical means for recording measurement data on a storage medium, for subsequent manual reading of such data, after the unmanned aerial vehicle of the system, equipped with the probe, has returned to the base. Preferably, the probe according to the invention includes an internal storage medium (SD card, SSD disk or the like) for recording the measurement values, the storage medium being located within the body of the probe, preferably near the other end of the body of the probe. Preferably, in such a configuration, the probe according to the invention is not wired to the unmanned aerial vehicle of the system according to the invention.

Preferably, the probe head according to the invention contains, in addition to the spectrometer or the measuring instrumentation cooperating with it (light sources, mirrors or lenses), at least one of the following: a moisture sensor, a soil conductivity sensor, a temperature sensor, an acidity sensor, while located inside the probe head, and its measuring electrodes are located on the outer surface of the probe body shell for allowing contact with the surface of the soil adjacent to the side of the body in the situation of embedding the probe in the tested soil.

Preferably, opposite to the first, the second end of the probe body contains technical means for the functional connection of the probe with the unmanned aerial vehicle of the system according to the invention, this connection being discussed in the section of the description on the unmanned aerial vehicle to which the probe according to the invention is coupled.

At the same time, it is obvious to a specialist in the field of technology that the measuring instrumentation used in the probe head is known and works according to known laws, just as the preferably used data carriers and electronic circuits, transmitters, receivers (together with control software) are essentially known from practice and the previous state of technology, which does not affect the essence of the solution with respect to the design of the soil probe according to the invention, facilitating the insertion of the measuring head essentially perpendicularly into the ground, allowing the screwing and unscrewing of the probe safe for wire connections, and also making it possible to carry out a soil property test along a helical path for covering as wide a range as possible of the ground surface adjacent to the probe sheath during its insertion into (as well as removal from) the ground, the advantages of the helical test path with the system's probe according to the invention being discussed in the section of the description on how to carry out a soil property test with the system comprising a probe according to the invention, coupled with the unmanned aerial vehicle of the system according to the invention.

The unmanned aerial vehicle of the system according to the invention, coupled with an in-ground probe according to the invention has a body provided with a known electrical power source, known electronic control and communication systems used in unmanned aerial vehicles (drones) adapted for communication with an external transmitter and an external receiver, and known technical means for setting the system in motion (propeller drive), whereby on the body of the unmanned aerial vehicle of the system is coupled a soil spectroscopy probe whose longitudinal axis is substantially perpendicular to the plane of the soil to be tested. Preferably, the probe is coupled to a dual-function linear-rotary drive mechanism, and the unmanned aerial vehicle of the system is preferably a flying drone of substantially known design.

In doing so, the linear-rotary drive mechanism is embedded on the body of the unmanned aerial vehicle of the system in such a way that the embedded probe has the ability to rotate around the longitudinal axis of the body and to move linearly in an essentially vertical axis, with the distance from the beginning of the probe blade according to the invention to the ground surface being selected in such a way that the distance from the blade to the ground surface is maintained in the situation of landing on the ground of the unmanned aerial vehicle of the system over the surveyed area.

Preferably, the linear-rotary drive mechanism, which realizes the rotary drive and the linear drive for the probe, is realized using independent drive systems. Preferably, the rotary mechanism, preferably of which the drive source is an electric motor, is separated from the linear mechanism and is preferably located near the centre of the unmanned aerial vehicle of the system.

Preferably, the advancing mechanism is realized by widely spaced actuators, preferably electric actuators, with preferably each actuator being within the end sections of the flying arms of the unmanned aerial vehicle of the system. Such spacing of the actuators allows control of the vertical position of the unmanned aerial vehicle of the system above the surface and enables penetration of the probing probe into the soil.

The considerable distance from each other and from the axis of rotation of the probe, the actuators, responsible for inserting and withdrawing the probe from the soil, increase the leverage effect and thus reduce the torsional torque acting on the advancing mechanism. Preferably, moreover, the actuators operate independently of both each other and of the rotary mechanism, and adjust (by means of a software-controlled change in their degree of extension) to the unevenness of the terrain in order to level the body of the unmanned aerial vehicle of the system, further increasing the stability of the entire system on the uneven ground tested with the object of the invention.

In addition, the rotational motion and the linear motion of the probe are independent of each other to eliminate the unfavourable tendency of the unmanned aerial vehicle of the system to be overturned (due to repulsion from the ground) by the probe embedded in the ground, due to encountering an underground obstacle during the probe's screwing in, or when the ground under investigation is hard. To determine the tilt value of the body of the unmanned aerial vehicle of the system coupled with the probe, the unmanned aerial vehicle of the system uses data readings preferably from the gyroscope, accelerometer and magnetometer included in the autopilot module or from a combination of readings from the sensors concerned. Beneficially, based on the data from the autopilot module, the unmanned aerial vehicle of the system adjusts the feed rate and speed of the probe.

The separation of the two ranges of motion allows the probe to be inserted into hard soil by exerting pressure perpendicularly in the direction of the soil, and to continue rotating the probe to slowly penetrate the soil until the probe blade reaches a depth of soil that allows the system according to the invention to stabilize and continue further penetration of the probe into the soil by screwing. A practical use of the above feature of the linear-rotary drive mechanism is the need to drill into heavily dried soil, the top layer of which forms a crust.

According to the invention, the linear-rotary drive mechanism seated on the body of the unmanned aerial vehicle of the system includes, conventionally in its lower part, a first holder for the probe body, preferably in the form of a clamp preferably rotatably bearing the probe body, cooperating in shape with the probe body according to the invention, whereby preferably on the lower plane of the clamp or on its inner surface are embedded technical means (for example: brush, gasket, scraping collar) for removing debris from the probe body during its movement through the clamp. In addition, the linear-rotary drive mechanism conventionally includes in its upper part a second holder for the probe body, the latter being slidably seated on a guide extending from the first holder toward the other end of the probe according to the invention, the second holder being in the form of a clamp containing technical means for imparting a rotary motion to the probe seated in the second holder.

For example, the second holder of the linear-rotary drive mechanism includes in the clamp a first electric motor and a toothed belt or drive chain coupling the shaft of the first motor to the toothed (serrated) section of the second end of the probe body according to the invention. Preferably, the imparting of rotary motion to the head is carried out by means of a toothed gear set in the second holder and coupling the shaft of the motor to the second end of the head body. Preferably, the first motor is provided with a gear.

In order to allow the probe to move in a substantially vertical axis for the purpose of plunging it or pulling it out of the ground to be tested, the second handle of the linear-rotary drive mechanism is slidably mounted on a guide which is substantially parallel to the longitudinal axis of the probe according to the invention and substantially perpendicular to the plane of the ground to be tested with the system and probe according to the invention.

The second handle of the probe's linear-rotary drive mechanism is seated on a carriage slidably seated on a linear guide, the linear guide being a profile rail on which, via bearing balls, the rolling carriage is slidably seated.

By convention, on the rear side of the carriage, opposite the probe body seated in the holders of the linear-rotary drive mechanism, the rolling carriage is provided with a ball nut cooperating in shape with a drive screw, preferably of trapezoidal thread, substantially parallel to the longitudinal axis of the probe body and the linear guide, whereby, in general, the overall length of the linear guide and the trapezoidal screw correspond to the length of the probe body less the length of the section of the body provided with the screw blade. The realization of the linear drive is carried out by translating the rotary motion of the trapezoidal screw coupled to the shaft of the second motor directly or through a coupling or gearbox, via a ball nut, into the linear motion of the rolling carriage along the linear guide, with the second motor conventionally located at the bottom of the linear-rotary drive mechanism, at its base.

In another variant, the linear guide for the carriage consists of two parallel rods of circular cross-section, parallel to each other and substantially parallel to the longitudinal axis of the probe, the rods being offset from each other and between them, substantially in the axis of the plane connecting the axes of the guides, there is situated, parallel to the rods, a screw - preferably with a trapezoidal thread, the length of which substantially corresponds to the lengths of the guides and which is the drive screw of the linear-rotary drive mechanism. By convention, at the bottom of the mechanism it is coupled to the shaft of the second motor, preferably by means of a coupling, preferably rigid. In addition, preferably the second motor comprises a gearbox. In doing so, the carriage moving along the slide is preferably connected by a trapezoidal threaded nut to a driving trapezoidal screw connected to the shaft of the second motor.

In doing so, the drive elements of the linear-rotary drive mechanism are connected by wire to the control system (an electronic system with control software) and a power source appropriate to the type of drive used in a known manner, with the drive power source and the control system embedded in the body of the unmanned aerial vehicle of the system.

In addition, the probe coupled with the linear-rotary drive mechanism of the unmanned aerial vehicle is in a configuration in which its components communicate by wire with components of the unmanned aerial vehicle of the system, is connected by wire to a device embedded on the body of the unmanned aerial vehicle of the system for analyzing the signal transmitted by wire from the probe head measurement instrumentation, whereby within the other end of the probe body the wires, which, depending on the configuration of the probe head, are electrical wires or optical fibres, is located another wire bearing point, preferably containing a rotatably arranged guide for the wires, in order to eliminate the unfavourable phenomenon of twisting of the wires during the rotary movement of the probe body, which would lead to permanent damage of the wires connecting the probe instrumentation with the device for analyzing the measurement signal. At the same time, it is obvious to the specialist that in the case of the probe according to the invention, whose instrumentation communicates with an unmanned aerial vehicle of the system components or an external computer wirelessly - it is devoid of the above solution, which does not affect the essence of the solution.

Preferably, the unmanned aerial vehicle of the system includes a device for analyzing the signal transmitted wirelessly from the probe's measurement instrumentation. In such a configuration, within the body of the probe, in addition to the measuring instrumentation, there are electronic circuits for wireless transmission of data from the measuring instrumentation to an external receiver.

Preferably, the measuring signal analysis device includes at least one spectrometer.

Preferably, the unmanned aerial vehicle of the system according to the invention contains at least one external light source.

Preferably, the unmanned aerial vehicle of the system according to the invention includes at least one image-recording camera. Preferably, the camera is a multispectral camera, whereby the image from the camera is preferably used by the software controlling the operation of the aerial vehicle of the system for making additional measurements of the state of the acreage on the basis of image analysis - for example, assessing the state of vegetation on the basis of the detected amount of chlorophyll in the image recorded in the visible light or infrared range, or determining the density of crops in given areas of the acreage. In addition, preferably, the image from the camera is used by the software controlling the operation of the system for the detection, on the basis of image analysis, of obstacles in the autonomous mode of operation of the unmanned aerial vehicle of the system or in the mode of control of the unmanned aerial vehicle of the system by the user.

Preferably, the unmanned aerial vehicle of the system according to the invention has the ability to determine the depth reached by the probe. For this purpose, it includes a proximity sensor, preferably a laser sensor or an ultrasonic sensor, located within the linear-rotary drive mechanism. Preferably, the system determines the depth position of the probe by measuring by laser or ultrasonic means the change in the distance between the first and second handles of the drive mechanism or between the carriage and the base of the linear-rotary drive mechanism, changing with the movement of the probe in the vertical plane, measuring by laser or ultrasonic means the distance between the second handle of the drive mechanism and the ground surface, changing with the movement of the probe in the vertical plane, or by measuring through a sensor implemented in the linear-rotary drive mechanism the number of revolutions of the first motor (preferably a rotary encoder), and an algorithm that converts the number of revolutions of the first motor in correlation with the pitch of the thread of the trapezoidal screw (if a guide of this type is used) to the position of the second handle with respect to the zero point (the maximum upward position).

Preferably, the unmanned aerial vehicle of the system according to the invention has software implemented that allows it to move, set the probe in motion, perform soil property surveys (control of the survey instrumentation), or create georeferenced maps in autonomous mode, with the unmanned aerial vehicle of the system preferably including a GPS sensor and a telemetry transmitter and receiver for enabling the assignment of survey data to geographic coordinates and survey depth values. Preferably, the creation of georeferenced maps is carried out in an external computer, equipped with software for processing the results of the survey, on the basis of the data sets acquired and transferred (or transferred from the internal data carrier) by the system according to the invention implementing the soil properties survey. The obtained georeferenced maps of the soil properties survey results, created on the basis of measurements with the probe coupled with the unmanned aerial vehicle of the system according to the invention, are then used in the planning of targeted tillage operations, contributing to the optimization of the consumption of materials and resources used in tillage operations.

Preferably within the body of the probe or within the feed drive mechanism or within the body of the unmanned aerial vehicle of the system, the solution according to the invention includes a calibration module having the form of a sedimentary pocket for a standard with a translucent part, whereby preferably the module is positioned so that the translucent part of the module is substantially tangential to the outer surface of the probe inspection hole, in a situation where the probe inspection hole is opposite the module, for enabling calibration with at least one spectroscope installed within the probe or within the body of the unmanned aerial vehicle of the system.

The method of conducting a soil property test according to the invention, implemented with the system according to the invention, consisting of an unmmaned aerial vehicle coupled with a probe , involves the delivery by the unmanned aerial vehicle of the system according to the invention of an in-ground probe coupled with an unmanned aerial vehicle to the measurement site. The probe is then introduced by a linear-rotary motion caused by the linear-rotary drive mechanism of the unmanned aerial vehicle of the system into the ground, delineating a cylindrical measuring section in the ground - extending from the surface of the ground to the place where the probe reaches a predetermined maximum depth, after which the direction of linear-rotary motion is changed and the probe is brought out of the ground, with at least one test of soil properties along the length of the measuring section, with the measurement following a helical trajectory.

Such a method of measurement carries a number of advantages that eliminate the inconveniences of the known state of the art, because the helical trajectory survey allows to perform, within a single penetration of the probe into the ground, measurements at different depths and in different directions with respect to the axis of the probe body.

In addition, there is an extension of the measurement path, especially compared to the measurement path used by the well-known driven in-ground probes, which allows a larger set of soil condition data to be generated during a single insertion of the probe into the ground, and therefore a more accurate determination of soil condition at the measurement site.

In addition, carrying out the test with a probe equipped with a blade reduces the risk of not being able to carry out the measurement if an underground obstacle (such as a stone) is encountered. For example, assuming by way of illustration that for one 360° rotation of the body, the probe is sunk into the ground by 2.5 cm, and it is defined to carry out a measurement every 5 cm of sinking into the ground (i.e., there are two rotations of the probe body per measurement interval), in the event of encountering an obstacle adjacent to the probe's body that makes it impossible to carry out a survey in a given direction - it remains possible to obtain survey results for the other directions on a given measurement interval.

Testing of soil properties, depending on the configuration of the measuring instrumentation installed inside the probe head according to the invention, includes - unrestrictedly, at least one of: spectroscopic testing (reflectance spectroscopy in the range of radiation from IR through VIS to UV - depending on the type of spectroscope and light source used either in the probe head or installed on the body of the unmanned aerial vehicle of the system) of soil parameters, testing of the electrical conductivity of the soil, testing of the pH of the soil, testing of the temperature of the soil, testing of the moisture content of the soil, with the test (or tests) being carried out before the probe is brought out of the soil.

Spectroscopic examination of the ground reflection spectrum is carried out by illuminating, through the probe's inspection hole, the ground surface tangent to the probe body with a beam of light emitted by its source (directly or through optical elements, for example: mirror, lens, optical fiber), collecting the light reflected from the ground surface tangent to the probe body through the probe's inspection hole (directly or through optical elements) and sending it (directly or through optical fiber) in real time to the spectrum analyzing device.

Preferably, the study of soil properties is conducted during the insertion of the probe.

Preferably, the study of soil properties is conducted while the probe is being brought out of the ground.

Preferably, for the duration of the soil properties test, the probe's linear-rotary motion is periodically interrupted, with the number and length of individual interruptions being determined before the start of the implementation of the method according to the invention, whereby the determination may be made by the user or may be specified in the software controlling the conduct of the test by the unmanned aerial vehicle of the system according to the invention in autonomous mode.

Preferably, the interruption of the probe's linear-rotary motion occurs at a predetermined depth of soil.

Preferably, the test of soil properties includes, in addition to the spectroscopic test, at least one measurement of at least one of the following: electrical conductivity of the soil, moisture content of the soil, temperature of the soil, acidity of the soil, while the test is carried out using sensors other than spectroscopic sensors built into the probe head according to the invention.

Preferably, testing of soil properties includes carrying out at least one reference measurement, the reference measurement being at least one of the measurements made with a non-spectroscopic sensor installed in the probe head according to the invention, the result of the reference measurement being the background for clarifying or eliminating ambiguities from the spectroscopic test.

Preferably, the reference measurement includes at least one measurement of at least one of: soil electrical conductivity, soil moisture, soil temperature, soil acidity.

Preferably, the reference measurement includes calibration, whereby the comparison of the result of the spectroscopic test, with a standard value (the so-called standard) is considered calibration. In doing so, the standard value is obtained on the basis of a spectroscopic examination of the standard (standard), which is a standardized sample of the relevant material.

For example, a reference measurement including calibration is carried out according to the following steps: before taking a series of measurements in a crop field by an unmanned aerial vehicle of the system according to the invention, coupled with a probe according to the invention, a measurement of the standard is carried out, after which the result of this measurement is transmitted wirelessly or wired to a results processing unit (computer, database server) equipped with software for processing the results of spectroscopic measurements.

A standard is a sample of material whose reflectance spectrum plot is known, which allows either the spectroscope or the software processing the spectroscopic test results to be properly calibrated to obtain the correct (according to the known standard) reflectance spectrum.

Before the soil condition test is performed, a spectroscope installed in the probe is used to measure the reflected spectrum of the standard. On this basis, the values of the divergence of the measurement made with the probe spectroscope from the known spectrum of the standard are determined. On the basis of the determined differences in the measurement with respect to the standard, the software processing the results of the soil test performs automatic calibration to make corrections to the result of the spectroscopic measurement.

Performing a calibration-based calibration of either the spectroscope or the software that processes the results of the spectroscopic measurement makes it possible to obtain more accurate (closer to the truth) graphs of the reflectance spectra of the spectroscopic soil survey after they are processed by the software.

Preferably, the reference measurement is used to calibrate the spectroscopic measurement result.

Preferably, calibration is carried out before the soil properties test.

Preferably, calibration is carried out after the soil properties test.

Preferably, during the implementation of the method according to the invention, the control software of the unmanned aerial vehicle of the system measures the current drawn by the first or second motor for determining the hardness of the ground into which the probe according to the invention is inserted based on the value of the current drawn.

Preferably, the measurement of the current drawn by the first or second motor is used to determine the moment of initial contact between the probe blade and the ground, resulting in an increase in the value of the current drawn by the motors - due to the resistance encountered by the probe blade associated with the start of the probe's penetration into the ground.

Moreover, preferably, the increase in the value of the deviation of the body of the unmanned aerial vehicle of the system from the horizontal, detected by the control software and determined for the unmanned aerial vehicle of the system by reading the parameters from the instrumentation of the autopilot module, is used by the control unit of the linear-rotary drive mechanism to correct the speed of the probe's advance by changing the rate or direction of rotation or linear motion of the motor or motors of the linear-rotary drive mechanism. In addition, during the implementation of the method according to the invention, the probe according to the invention is embedded in the feed drive mechanism of the unmanned aerial vehicle of the system according to the invention.

Preferably, in the implementation of the method according to the invention, more than one unmanned aerial vehicle of the system equipped with a probe is used. In such a method of performing a soil property testing sequence, the unmanned aerial vehicle of the system perform simultaneous testing of one or more cultivated acres, whereby, as is obvious to the specialist, each system, independently of the others, performs the testing in accordance with the disclosure, which does not affect the essence of the invention but is a further advantageous development of the invention that allows reducing the duration of soil property testing on a given acreage.

It is evident to the specialist that, with the increase in the number of the unmanned aerial vehicles of the system implementing the method according to the invention, the control and communication with the unmanned aerial vehicle of the system and the measurement instrumentation contained within them or within the probe head is based on analogous principles of communication and control as in the case of the use of a single unmanned aerial vehicle of the system, with these technical and functional features described more extensively with respect to a single unmanned aerial vehicle of the system equipped with a probe and implementing the method according to the invention.

In addition, preferably, the unmanned aerial vehicle or vehicles of the system start the soil properties testing sequence by taking off from a mobile launch platform, from which they move towards the measuring points in a given cultivation field (or in given fields in case more than one system is used to test soil properties in more than one cultivation field in parallel).

After completing the sequence of testing the soil properties of an acreage (or acres), i.e. after completing the last survey sequence at the last designated point of the surveyed acreage, the unmanned aerial vehicle of the system or systems remain within the surveyed acreage, and when the mobile launch platform comes within their range again, they return to the mobile launch platform, whereby the launch, the sequence of testing the soil properties of an acreage, and the return to the mobile launch platform are preferably carried out in autonomous mode.

When carrying out a survey of soil properties in different crop fields, a ground vehicle, preferably a vehicle with a mobile launch platform of unmanned aerial vehicle of the system, travels on roads between crop fields. Once the vehicle is within range of the crop field where the survey is to be carried out, the unmanned aerial vehicle of the system assigned to carry out the survey takes off from the mobile platform, preferably autonomously moves to the first survey point on the acreage in question and begins surveying the acreage in question. Then, after the survey is completed, the unmanned aerial vehicle of the system waits at the surveyed acreage until the ground vehicle with the mobile platform comes within range of the unmanned aerial vehicle of the system again and returns preferably autonomously aboard the mobile platform.

When carrying out a survey of soil properties using at least one unmanned aerial vehicle of the system on a single cultivation field, the unmanned aerial vehicles of the system take off from a ground vehicle containing a mobile launch platform and moving on or near the cultivation field. Once the measurement points come into range, the unmanned aerial vehicle of the system assigned to the measurement point, preferably autonomously, takes off from the mobile platform, moves to the first measurement point and begins taking measurements on the acreage. After taking a certain number of measurements, the drone remains on the given areal and waits until the ground vehicle is again within range to return to the mobile launch platform.

Preferably, the unmanned aerial vehicle of the system returning to the mobile launch platform undergoes an operation to recharge its battery before being delivered to a new area to be tested according to the invention.

Preferably, the mobile launch platform can be implemented not only on known automotive vehicles but also on known agricultural vehicles (tractors, harvesters).

Preferably, the results of the survey conducted with the unmanned aerial vehicle of the system are applicable to planning precision tillage treatments with other types of autonomous agricultural machinery, such as autonomous fertilizer equipment, which preferably includes mobile launch platforms. Such a solution makes it possible to carry out precise fertilizer treatments based on field condition data generated by the unmanned aerial vehicle of the system in real time while conducting a soil condition survey.

Preferably, unmanned aerial vehicles of the system either operate in autonomous mode or operate on the basis of commands issued by the operator (user) of unmanned aerial vehicles of the system.

The subject of the application fully realizes the technical issue posed to it by eliminating the inconveniences known from the state of the art. The individual components grouped together as part of the soil properties testing system containing an unmanned aerial vehicle coupled to a probe synergise to meet the technical challenge posed by the invention. An in-ground probe provided with technical means for facilitating the insertion and removal of the probe from the ground by screwing it in and out of the ground using at least one projection, preferably in the form of a blade, the principal surface of which is at least partially contained within the screw surface determined around the longitudinal axis of the probe allows the reduction of the perpendicular pressure force during the insertion of the probe into the ground, and thus makes it possible to reduce the dimensions and masses of the elements of the linear-rotary drive mechanism, which in turn makes it possible to introduce the probe into the ground and perform soil testing with a relatively lightweight unmanned aerial vehicle of the system coupled with the probe, without the risk of causing the probe carrier to tip over.

In addition, the design of the probe incorporating a screw blade and moving in a rotary and linear manner enables the testing of soil properties along a helical trajectory, which in turn allows measurements to be carried out at different depths and in different directions relative to the probe body, extends the measurement path allowing more measurement data to be collected at a given point in the surveyed area and soil parameters to be determined based on a broader set of information. In addition, the helical blade of the probe moving rotationally and linearly reduces the risk of blocking the measurement at a given measurement point due to encountering an underground obstacle that would prevent further advance of the driven probe. What's more, measuring along a helical trajectory makes it possible to carry out a correct soil measurement at a given depth interval when the probe encounters an underground obstacle tangent to the probe body and obscuring the head inspection hole.

In addition, coupling the probe with an unmanned aerial vehicle of the system eliminates the need for the user to be directly involved in driving the probe in and out of the ground.

What's more, the use of multiple unmanned systems consisting of unmanned aerial vehicles, eachcoupled with a probe according to the invention makes it possible to reduce the time it takes to test the soil properties on a given cultivation area.

The use of a ground vehicle traveling on roads, between surveyed fields, and equipped with a mobile launch platform for unmanned aerial vehicle(s) of the system - increases the operational range of a group of unmanned aerial vehicle(s) of the system, and makes it possible to optimize the time required to conduct surveys on several crop fields. For example, having a database of users and the coordinates of their crop fields, it is possible to select the optimal route for a ground vehicle, equipped with a mobile launch platform for the unmanned system(s), to travel between these crop fields to perform surveys.

In addition, the use of a ground-based vehicle moving through agricultural fields allows for increased coverage of unmanned aerial vehicle(s) of the system both by being able to transport the unmanned aerial vehicle(s) of the system and by being able to charge the batteries of the unmanned aerial vehicle(s) of the system on a mobile platform located on the vehicle. In addition, this allows integration with state-of-the-art autonomous precision farming machines, such as precision fertilization machines - the results of the unmanned systems can be used in real time by these machines to perform targeted tillage operations.

The object of the invention is shown in the attached drawing, where:
- Fig.1 shows a longitudinal cross-section of the measuring part of the in-ground probe according to the invention in the first example of implementation,
- Fig.2 - a longitudinal cross-section of the measuring part of the in-ground probe according to the invention in the second example of implementation,
- Fig.3 - a longitudinal cross-section of the measuring part of the in-ground probe according to the invention in the third example of implementation,

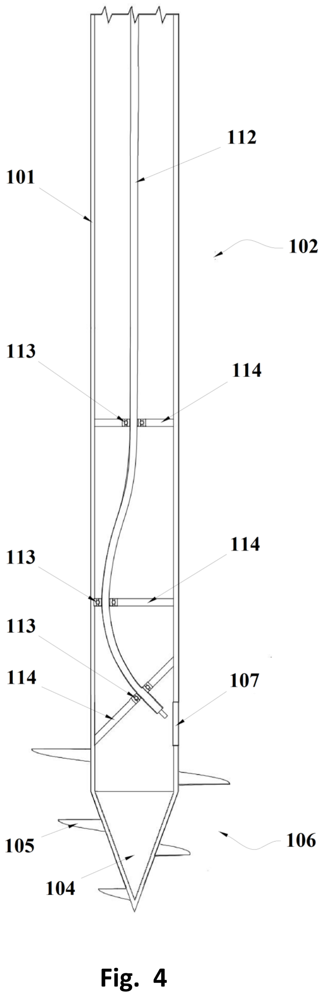

- Fig.4 - a longitudinal cross-section of the measuring part of the in-ground probe according to the invention in the fourth example of implementation,
- Fig.5 - unmanned aerial vehicle of a system coupled with a probe, in side view, in the first example of implementation,
- Fig.6 - axonometric view of the system from fig.5,

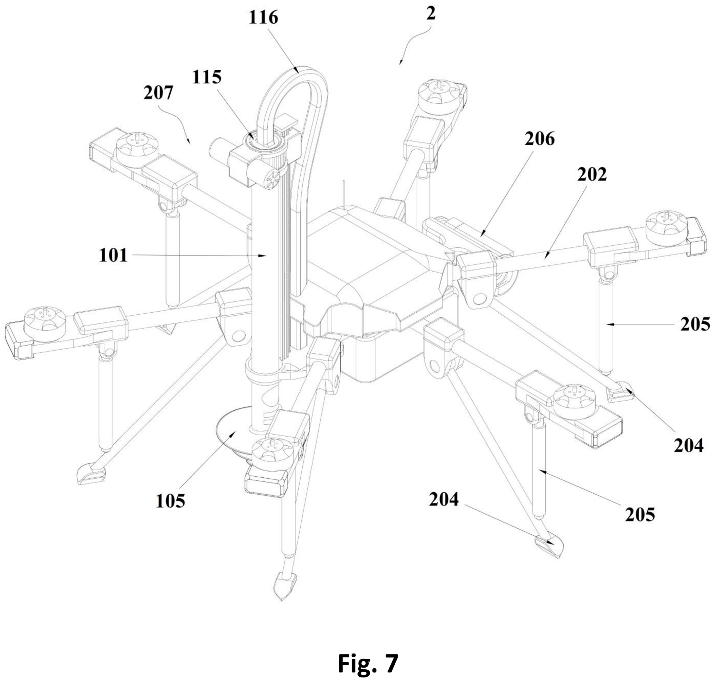

- Fig.7 - axonometric view of the system from fig.5, from the side of the probe, with the propellers removed,
- Fig.8 - axonometric view of the system in the second example of implementation, from the bottom side,
- Fig.9 - side view of the linear-rotary drive mechanism in the first example of implementation with the probe according to the invention embedded in it,
- Fig.10 - axonometric view of the linear-rotary drive mechanism in the first example of implementation with the probe according to the invention embedded in it.

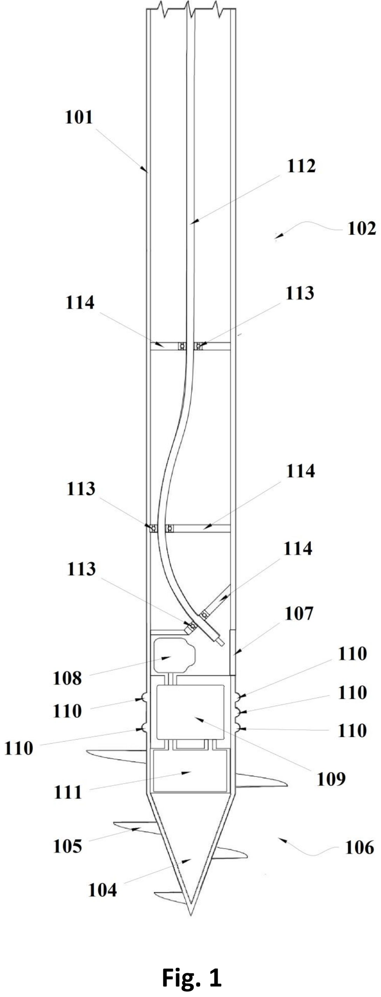

In the first example of implementation illustrated in Fig.1 the in-ground probe 1 adapted to being coupled with an unmanned aerial vehicle of the system, according to the invention has a tubular body 101 which has a measuring part 102 and a settling part 103 opposite thereto, the measuring part 102 of the body 101 ending conventionally at the bottom with a tapered first end 104, whereby on the side of the first end 104 and conventionally the lower part of the measuring part 102 of the body 101 is a projection having the form of a blade 105, with the basal surface of the blade (105) contained within a helical, rectilinear surface defined around the longitudinal axis of the probe 1.

Conventionally, in the lower measuring part 102 of the body 101 of the probe 1 is a head 106, whereby in the side of the body 101, within the head 106, an inspection hole 107 protected by sapphire glass is formed and, moreover, inside the body 101, within the head 106, the elements of the measuring system are located: a light source 108, whereby the light source 108 is located opposite to and facing the inspection hole 107. In addition, conventionally below the light source 108, the head 106 includes a sensor assembly 109 for measuring soil moisture, soil temperature, acidity and soil conductivity, the measuring electrodes 110 of which extend outside the body 101 of the probe 1. In addition, conventionally below the sensor assembly 109, within the head 106 is a battery 111, functionally connected to supply electricity to the light source 108 and the sensor assembly 109.

In addition, inside the body 101 of the probe 1 is located an optical fiber 112 which is rotatably arranged in bearing points 113, which are plain bearings, embedded in partitions 114, whereby within the head 106, the free end of the optical fiber 112 is directed toward the inspection hole 107 for allowing the collection and transmission of a spectrum of light reflected from the surface of the soil under test tangent to the inspection hole 107. Opposite the head 106, the optical fiber 112 is arranged rotatably in a swivel joint 115 located in the settling part 103 of the body 101 of the probe 1 and further runs toward a spectrometer located within the body 201 of the unmanned aerial vehicle 2 of the system via a guide 116.

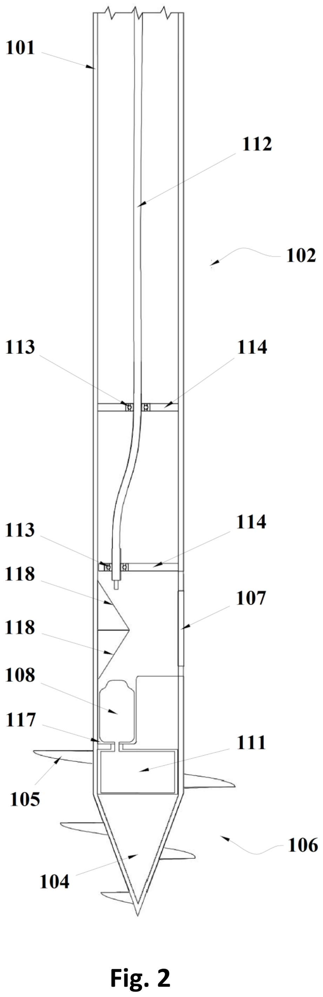

In the second example of implementation shown in Fig.2, the in-ground probe 1 adapted to being coupled with an unmanned aerial vehicle of the system, has a tubular body 101 having a measuring part 102 and a settling part 103, a tapered first end 104 provided with a blade 105, with the measuring part 102 containing a head 106 having an inspection hole 107 protected by quartz glass. Within the head is located in a socket 117 a light source 108 powered by a rechargeable battery 111, the light source being conventionally located below the lower edge of the inspection hole 107. In addition, within the head are located two mirrors 118, the planes of which are angled toward each other in such a way that the contact point of the surfaces of the mirrors is located in the axis of the inspection hole, and the free ends of the mirrors are in contact with the inner surface of the body 101 of the probe 1. In doing so, the angle between the mirrors 118 is selected so as to direct the light beam from its source 108 into the inspection hole 107 and the light beam reflected from the ground layer adjacent to the inspection hole 107 is directed toward the free end of the optical fiber 112, rotatably supported in bearing points 113 embedded in the baffles 114, the bearing points 113 being rolling bearings. In the example implementation, the free end of the optical fiber 112 is located conventionally near the upper mirror 118 and, opposite the head 106, the optical fiber 112 is rotatably borne in a swivel joint 115 located in the settling part 103 of the probe body 101 1 and further runs toward a spectrometer located within the body 201 of the unmanned aerial vehicle 2 of the system via a guide 116.

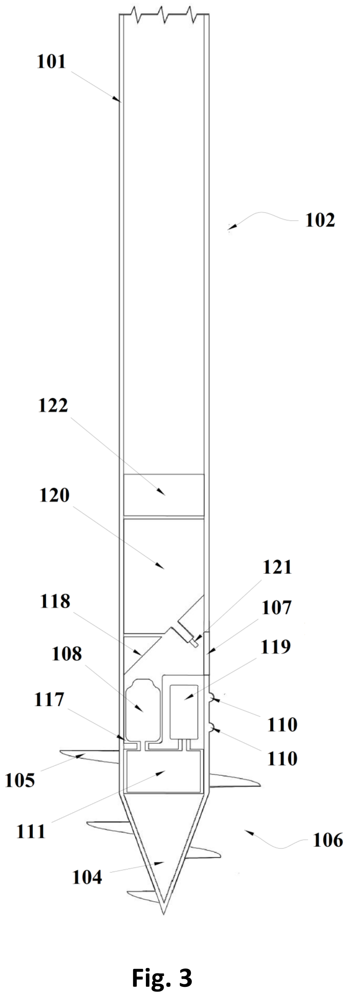

In the third example of implementation, shown in Fig.3, in-ground probe 1 adapted to being coupled with an unmanned aerial vehicle of the system, having a tubular body 101 of analogous construction as in the first and second examples of implementation, has within the head 106 a light source 108 and a moisture sensor 119, the measuring electrodes 110 of which extend outside the body 101 of probe 1. In addition, in the head 106 conventionally above the light source 108 is located a mirror 118, directing the light beam from its light source 108 to the inspection hole 107. Furthermore, conventionally above the light source 108, in the head 106 of the probe is located a spectrometer 120, the entrance slit 121 of which is located near the inspection hole 107. In doing so, the light source 108, the moisture sensor 119 and the spectrometer 120 are supplied with electricity from a battery 111 conventionally embedded in the lower part of the probe head 106. In this example implementation, the spectrometer 120, the light source 108, and the moisture sensor 119 are communicated wirelessly, via a wireless communication module 122, with a control unit within the unmanned aerial vehicle 2 of the system, whereby the wireless communication module 122 includes a transmitter, a receiver, and an electronic circuit that controls the various functional components of the probe based on commands received wirelessly from the unmanned aerial vehicle 2 of the system.

In the fourth example implementation, the in-ground probe 1 adapted to being coupled with an unmanned aerial vehicle of the system, has a tubular body 101 having a structure analogous to that of the preceding examples of implementation, with a free end located within the head 106, rotatably arranged in bearing points 113 embedded in baffles 114, of an optical fiber 112 providing a light beam to the inspection hole 107 from a light source located outside the perimeter of the probe 1, and also transmitting a beam of light reflected from a ground surface adjacent to the inspection hole 107 toward a spectrometer 120, the light source and spectrometer being installed within the body 201 of the unmanned aerial vehicle 2 of the system, with the optical fiber 112 opposite the head 106 bearing rotatably in a swivel joint 115 located in the settling part 103 of the body 101 of the probe 1 and continuing to run toward the body 201 of the unmanned aerial vehicle 2 of the system via a guide 116. In this example of implementation, the optical fiber 112 is a bundle of optical fibres of the type 6-window-1, i.e. the six optical fibres surrounding the central reading fiber are illuminating optical fibres functionally connected to a light source, whereby, as is obvious to the specialist - it is possible to use bifunctional optical fiber bundles with a different configuration of optical fiber strands.

What is obvious to the specialist of the field of technology, in further examples of implementation of the invention, the probe 1 adapted to being coupled with an unmanned aerial vehicle of the system, within the head 106 includes more than one sensor, the measuring electrodes 110 of which extend outside the body 101 of the probe 1. Furthermore, in further examples of implementation, the head 106 includes more than one light source 108. In doing so, it is obvious to the specialist that the light source(s) 108, are light sources emitting light of different spectra: white light, infrared light, ultraviolet light, whereby, depending on the example implementation, the head 106 of the probe 1 contains one or more light sources 108, the spectra of which are analysed by at least one spectrometer 120, with additional spectrometers located within the head 106 or within the unmanned aerial vehicle 2 of the system. What is also obvious to the specialist, in further examples of the implementation of the probe 1 according to the invention, the optical element directing the light beam to or from the inspection hole 107 may be, in place of the mirror 118, a lens, mirror or other optical element affecting the direction, focus or dispersion of the light beam emitted by its light source 108 - which does not affect the essence of the invention but only constitutes further advantageous examples of its implementation.

In still further examples of implementation, within the body of the 101 probe is located an electronic circuit for processing and recording data acquired from measuring instruments (spectrometer 120, sensors) on an SD-type memory card.

In still further examples of implementation, more than one protrusion is located on the outer surface of the body 101, with the essential surfaces of the protrusions contained within a surface defined between a curve rotating at a constant speed around the longitudinal axis of the probe 1 body 101 and moving parallel to this axis at a constant linear speed.

In still further example of implementation, the spectrometer 120 and light source 108 are embedded in the body 201 of unmanned aerial vehicle 2 of the system, being functionally connected via optic fibre cables with the head 106 of the probe 1. What is obvious to those skilled in art, is that in this embodiment, light source 108 emits light which is transferred via optic cable to the inspection hole 107 of the head 106 and spectrum reflected by the ground is transferred via optical fibre back to the spectrometer 120 embedded in unmanned aerial vehicle 2 of the system. What is also obvious to those skilled in art, is that it is possible to embed more vital elements of the probe head 106, like power sources, memory modules, sensors described in previous embodiments, spectrometers, and light sources, inside unmanned aerial vehicle 2 of the system and functionally connect (via optic fibres or electrical cables) these features with the probe head 106 or inspection hole 107, or electrodes embedded on the outside of the probe 1 head 106. These further embodiments should not be treated as restrictive but as further, yet advantageous embodiments of the invention.

It is obvious to the specialist that in further examples of implementation, it is possible to communicate and wire power the components of the head 106 of the probe 1, including the sensor assembly 109, the light source 108 and the spectrometer 120 with the control elements, collecting data and processing them and implemented within the body 201 of the unmanned aerial vehicle 2 of the system - analogously to the examples of implementation involving cables bearing in the body 101 of the probe 1, running through the swivel joint 115 and through the guide 116 to the unmanned aerial vehicle 2 of the system.

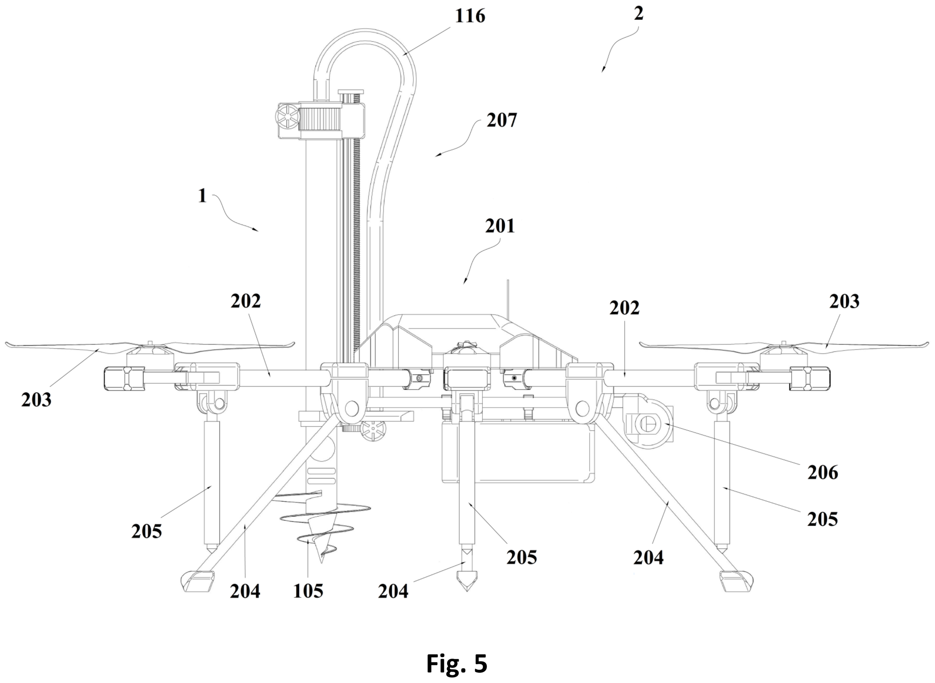

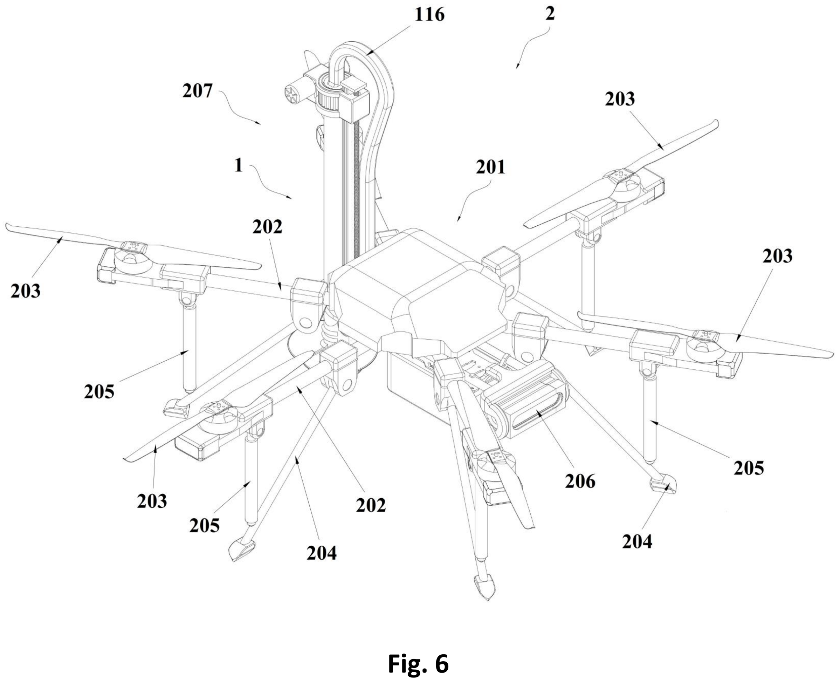

Fig.5 and Fig.6 reveal the unmanned aerial vehicle 2 of the system, adapted to being coupled with an in-ground probe 1, in the first example of implementation, which is a flying propeller drone. The unmanned aerial vehicle 2 of the system has a body 201 to which arms 202 containing electrically driven propellers 203 at their free ends are connected. In addition, the arms 202 are connected to the legs 204 via shock absorbers 205. It is obvious to the specialist that within the body 201 the unmanned aerial vehicle 2 of the system includes devices and systems for flight control (electronic controllers, central unit), multispectral camera 206, components for orientation in space (GPS module, autopilot module including gyroscope, accelerometer and magnetometer) and wireless communication (receiver, transmitter, antenna) with the operator of the unmanned aerial vehicle 2 of the system or with an external computer unit supervising the parameters and functions of the unmanned aerial vehicle 2 of the system, as well as electronic circuits controlling the operation of the functional elements of the probe 1 and the mechanism of the linear-rotary drive mechanism 207, and a built-in source of electrical energy (battery), with the battery located on the body 201 of the unmanned aerial vehicle 2 of the system from its underside.

A linear-rotary drive mechanism 207 is mounted on the body 201 of the unmanned aerial vehicle 2 of the system, in which a probe 1 according to the invention is embedded, whereby the linear-rotary drive mechanism 207 is wired to a power source embedded within the body 201 and furthermore the linear-rotary drive mechanism 207 is wired for communication with a control system embedded within the body 201, which controls the movement of the functional elements of the linear-rotary drive mechanism 207.

What is also obvious to the specialist, in both the first and subsequent examples of implementation, the unmanned aerial vehicle 2 of the system 2 is provided with software that controls both the manner and extent of movement of the system 2 and the operation of its various components, including the linear-rotary drive mechanism 207.

In both the first and subsequent examples of implementation of the unmanned aerial vehicle 2 of the system, the control unit communicates by wire or wireless, depending on the configuration of the probe 1, with the measurement instrumentation located in the probe 1, or in the head 106 of the probe 1, or at least partially embedded in unmanned aerial vehicle 2 of the system and functionally connected with the head 106 of probe 1, and controls the operation of these instruments (sensors, light sources), and in the examples of implementation, in which at least one spectrometer is located in the probe 1, in the head of the probe 1 or at least is partially embedded in unmanned aerial vehicle 2 of the system and functionally connected with the head 106 of probe 11, controls the operation of the spectrometer and accepts the signal, generated from the spectroscopic measurement of the light beam reflected from the ground surface adjacent to the inspection hole 107 of the probe by the spectrometer 120, for its further analysis.

In further examples of implementation, the body 201 of the unmanned aerial vehicle 2 of the system includes at least one spectrometer or at least one light source, whereby, depending on the particular example of implementation, the light sources and the spectrometers are functionally communicated with the inspection hole 107 of the probe 1 via a bundle of optical fibres reaching the probe body 101 via a guide 116 rotatably arranged in the second end of the probe body 101 of the probe 1. In such an example of implementation, the aerial vehicle 2 of the system has been coupled with the linear-rotary drive mechanism 207 the probe 1 according to the fourth example of its implementation, or a probe 1 of related design as presented in the fourth example of its implementation.

At the same time, it is obvious to the specialist that when the body 201 of the unmanned aerial vehicle 2 of the system contains only at least one spectrometer and no light source, the probe 1 contains at least one light source 108 in the head 106. In addition, it is obvious that in order to maintain the full functionality of the unmanned aerial vehicle 2 of the system in combination with the probe 1 for the implementation of the method of testing soil properties according to the invention - different (mixed) configurations are possible in still further examples of implementation, where some of the light sources and spectrometers are within the body 201 of the unmanned aerial vehicle 2 of the system and other light sources 108 and spectrometer 120 (or spectrometers) are embedded within the head 106 of the probe 1, which does not affect the essence of the disclosure but only provides further advantageous ways of implementing the invention. In doing so, it is obvious to the specialist that these functional elements can be communicated with the unit controlling them either wired or wirelessly, and are, depending on their embedding location, powered either from the battery 111 of the probe 1 or from the battery of the unmanned aerial vehicle 2 of the system.

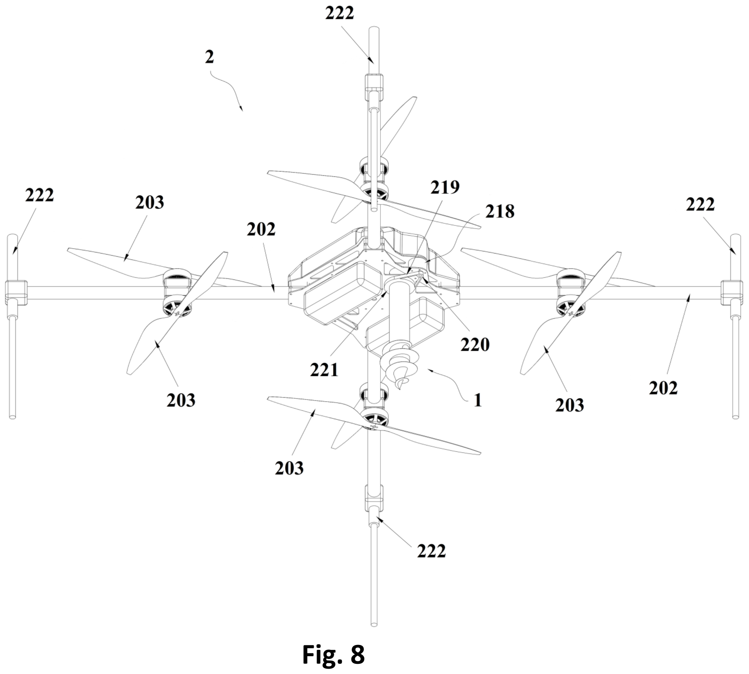

In an further example of implementation, shown in fig.8, the unmanned aerial vehicle 2 of the system, which is a propeller-driven flying drone, has a linear-rotary drive mechanism 207 formed of two independent drives, working together and controlled by the control software, independently of each other. The first drive within the linear-rotary drive mechanism 207 is realized by a rotary mechanism 218, using an electric motor with a shaft aligned parallel to the body of the probe 1. The electric motor's torque is transmitted to the body 101 of the probe 1 through a drive belt 219, seated on one side on a first pulley 220 located on the shaft of the electric motor and on the other side on a second pulley 221 conventionally integrated into the upper end of the body 101 of the probe 1, with the probe 1 being rotatably seated in the body 201 of the unmanned aerial vehicle 2 of the system. The second drive, as part of the linear-rotary drive mechanism 207, is carried out by a linear mechanism using a system of actuators 222, located one at the end of each of the arms 202, the actuators 222 being electric actuators operating independently of each other to enable the body 201 of the unmanned aerial vehicle 2 of the system to be levelled on the uneven ground on which the unmanned aerial vehicle 2 of the system is operating during the performance of the spectroscopic survey with the probe 1. In doing so, it is clear that the direction of movement of the actuators 222 is substantially perpendicular to the plane of the ground, enabling the up-and-down movement of the body 201 of the unmanned aerial vehicle 2 of the system.

In yet another embodiment, where at least one of the following: a spectrometer 120 and/or light source 108 is embedded inside the body 201 of unmanned aerial vehicle 2 of the system, the body 101 of the probe 1 is rotatably driven by the linear-rotary drive mechanism 207, connected to the body 101 of probe 1 via drive belt 219, while the spectrometer 120 and/or light source 108 remain stationary in relation to the rotary movement of probe 1. Optic fibres are connecting them functionally with the inspection hole 107 of the head 106 of the probe 1. It is obvious to those skilled in the art, that in these embodiments, optic fibres are not rigidly connected to the inner shell of body 101 of probe 1. It is also obvious, that in these types of embodiments according to the present invention, inspection hole 107 of the probe 1 can be an annular, transparent part of the probe 1 head 106 shell.

What is also obvious to those skilled in the art is that the embodiments mentioned herein, whether based on the newly described embodiment or any previously detailed embodiment, should not be viewed as restrictive. Instead, they represent additional, yet advantageous options for further implementations of the present invention., especially embodiments that cover other vital elements, like battery, wireless communication modules etc., are embedded at least partially in the body 101 of probe 1 or in body 201 of unmanned aerial vehicle 2 of the system.

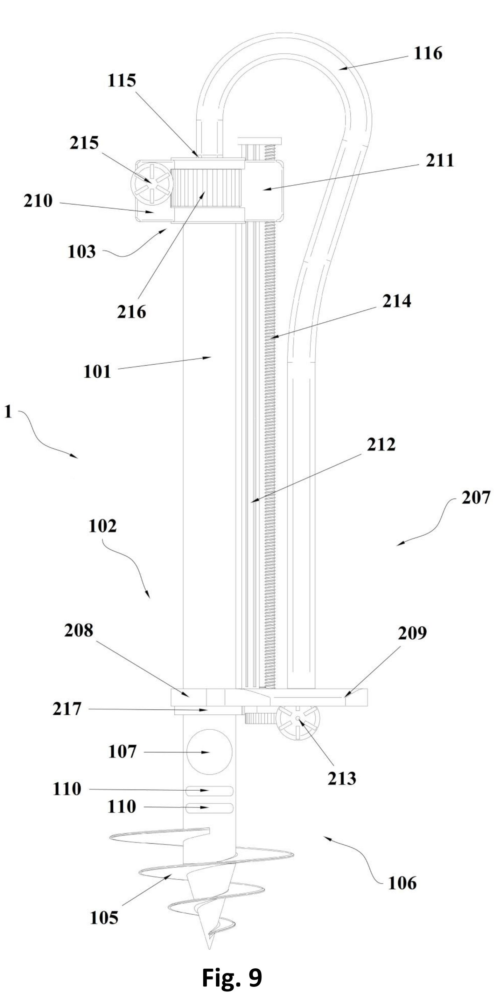

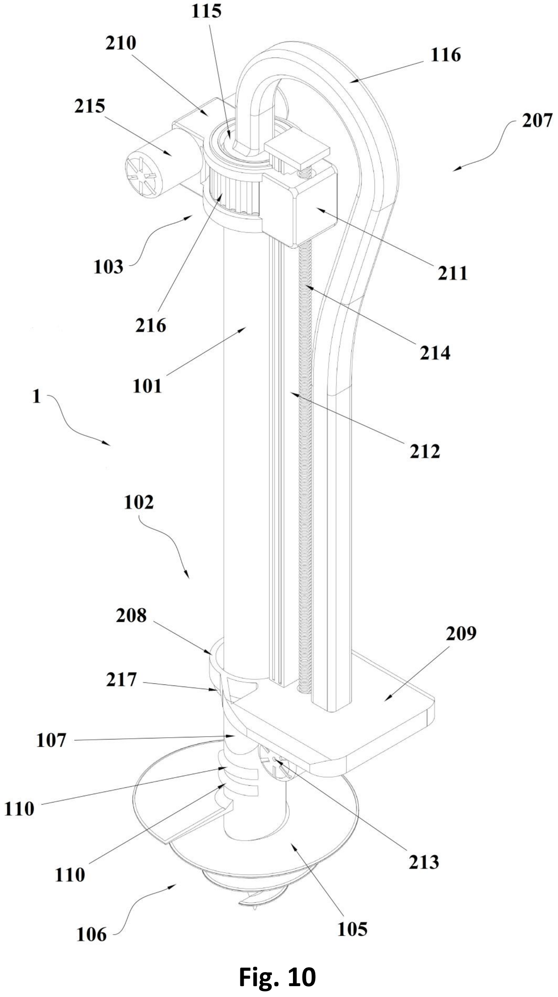

The linear-rotary drive mechanism 207, in another example of its implementation shown in fig.9 and fig. 10, includes a first handle 208 conventionally located at the bottom of the linear-rotary drive mechanism 207 and having the form of a clamp for the body 101 of the probe 1, the first handle being unilaterally fixed on the base 209. In addition, the linear-rotary drive mechanism 207 has a second handle 210 having the form of a clamp for the body 101 of the probe 1, the second handle 210 being unilaterally fixed on a carriage 211 slidingly mounted on a linear guide 212. In doing so, the linear guide 212 is substantially parallel to the body 101 of the probe 1 seated in the first 208 and second 210 handles of the linear-rotary drive mechanism 207 and is conventionally connected at the bottom to the base 209, the base 209 being substantially perpendicular to the base 209.

To set the probe 1 into linear motion, the linear-rotary drive mechanism 207 has the first motor 213 embedded in the base 209, the shaft of which is connected to the screw 214, which is parallel to the linear guide 212 and has a trapezoidal thread, while to translate the rotary motion of the screw 214 into the linear motion of the carriage 211, it is provided with a ball nut that cooperates with the thread of the screw 214.

In addition, the second handle has, located opposite the carriage 211, a second motor 215 connected by a gearbox to the body 101 of probe 1, provided with a longitudinal serration 216 for coupling to the gearbox of the second motor 215 for putting the body 101 of probe 1 into rotary motion.

In addition, to remove debris from the body 101 of the probe 1, the first handle 208 is provided with a scraping ring 217.

In the second example of realization of the linear-rotary drive mechanism 207, the carriage 211 is seated on two parallel rod-like guides 212, between which is located a screw 214 set into rotary motion by the first motor 213. In this example of implementation, the body 101 of the probe, provided with a longitudinal serration 216, is coupled to the shaft of the second motor 215 via a toothed drive belt and the element that cleans the body 101 of the probe 1 from soil residue is a brush seal, set omnidirectionally with respect to the through hole of the first handle 208.

In a further example of the implementation of the linear-rotary drive mechanism 207, a laser distance sensor is located within the carriage 211, measuring the distance between the second handle 210 and the base 209 of the linear-rotary drive mechanism for determining the depth of insertion of probe 1 into the ground.

In another example of implementation, a shaft rotation sensor in the form of a rotary encoder is embedded on the shaft of the first motor 213, between its housing and the point of connection to the shaft of the screw 214, for measuring the number of revolutions of the shaft of the first motor 213 and thereby determining the depth of insertion of probe 1 into the ground.

In yet another example of implementation, an ultrasonic sensor is embedded on the second handle 210 of the linear-rotary drive mechanism 207, which measures the distance between the second handle 210 and the ground level, for determining the depth of insertion of probe 1 into the ground.

In yet another example of the implementation of the linear-rotary drive mechanism 207, the body 101 of probe 1 is functionally connected, via a longitudinal serration 216 to a worm mounted on the shaft of a second motor 215.

The method of conducting a test of soil properties carried out by the system comprising an unmanned aerial vehicle 2 coupled with a probe 1 according to the invention, in which the linear-rotary drive mechanism 207 embeds the probe 1 according to the invention, in the example of implementation involves moving the unmanned aerial vehicle 2 of the systemcoupled with the probe 1 to the place of measurement on the surveyed area, determining by the user the depth interval of the test (the depth range in which the system comprising an unmanned aerial vehicle 2 coupled with a probe 1will return measurement results), where the test interval is understood as parts of the measurement section (i.e. the section from the ground surface to the point of maximum depth of taking the measurement), specified by the user (for example: on a 15 cm measuring section, a 5cm depth test interval is specified, i.e. the measuring section is divided into ranges: 0 to 5, >5 to 10, >10 to 15 cm - constituting survey intervals). In addition, in further examples of implementation it is allowed, the determination of several survey intervals, not necessarily of equal length and at equal intervals.

The unmanned system comprising an unmanned aerial vehicle 2 coupled with a probe 1 then brings the probe 1 in a linear motion to the point where the end of its blade 105 comes into contact with the ground surface, after which the system 2 starts the second motor 215, putting probe 1 in a rotating motion, after which the downward linear motion of probe 1 continues by the system comprising an unmanned aerial vehicle 2 coupled with a probe 1 starting the first motor 213 resulting in the gradual penetration of probe 1 into the ground.

Then, inside each user-specified test interval, the control unit of the system sends once a control signal for the generation of a light pulse by the light source 108 and a spectrometer 120 activation signal for performing a spot reflection spectrum analysis of the ground surface tangent to the inspection hole 107 of the body 101 of the probe 1. At the same time, the control unit of the system comprising an unmanned aerial vehicle 2 coupled with a probe 1 sends a signal activating the sensor assembly 109 (or moisture sensor 119, or other [non-spectroscopic] sensor or sensors provided for use in the head 106 of the probe 1 according to the invention - depending on the example of the implementation of the probe 1). The data acquired by the control unit from the spectrometer and sensor(s) are then stored in the memory of the control unit (or on an SD-type memory card - depending on the example of the implementation of the probe 1 according to the invention, or transmitted wired/wireless to the control unit of the unmanned system comprising an unmanned aerial vehicle 2 coupled with a probe 1), whereby the immersion of the probe 1 into the ground is interrupted for the duration of the measurements at a given interval and, after the measurements are made, is continued until the probe 1 reaches the maximum depth of the survey (reaching the lower end of the survey section). The sequence of activation of the measuring components of the head 106 is repeated cyclically until the probe 1 reaches the maximum depth, after which the direction of linear motion and the direction of rotary motion of the linear-rotary drive mechanism 207 are reversed and the probe 1 is brought out of the ground, after which the unmanned system comprising an unmanned aerial vehicle 2 coupled with a probe 1 moves to the next location of conducting the survey and repeats the sequence of actions according to the first example of implementation of the method according to the invention.

In the second example of implementing the test method, based on the first example, each of the measurement sequences is carried out continuously at specific intervals indicated by the user. For example: with a 5 cm interval for a 15 cm test section, continuous measurements are carried out at intervals of 0-5 cm and >10 to 15 cm, with the >5 to 10 cm test interval omitted, and for the duration of the continuous measurement, the movement of probe 1 is not stopped and the measurement is a continuous measurement at the given interval. The sequence of movement of probe 1 and the measurement sequences continue until probe 1 reaches the maximum measurement depth, after which unmanned aerial vehicle 2 of the system changes the direction of linear movement and the direction of rotary movement of probe 1 until it is brought out of the ground, after which the unmanned aerial vehicle 2 of the system equipped with probe 1 moves to the next measurement point in the surveyed area and repeats the survey method according to the example until the last survey is performed at the last point in the surveyed area.

As is obvious to a specialist of the field of technology, in further examples of the implementation of the method, the measurement sequence can be performed continuously over any predetermined length of the measurement path, where the length of the measurement path is understood to be the length of the helical segment traversed by the inspection hole 107, and this length can be shorter than the length of the entire helical segment traversed by the inspection hole 107 at a given interval.

In the next example, based on the previous example, the method of testing involves cyclically repeating a sequence of stops, measurements and resumptions of probe 1's movement as it moves out of the ground.

In the following example of the implementation of the test method, based on the first or second example of the implementation of the test method, two measurement sequences are carried out in a given test interval, where, in the example based on the first example of the implementation of the test method, in a given test interval, the number of sequences of interruptions and resumptions of probe 1 movement is equal to the number of measurement sequences.

It is obvious in the light of the present disclosure that, in further examples of the implementation of the method of testing according to the invention, a number of measurement sequences greater than two is permitted in a particular interval of testing, for collecting as large a sample of soil test results as possible at a particular interval of testing. Also evidently, in the case of the implementation of the method comprising sequences of interruptions and resumptions of movement of the probe 1, the number of such sequences is equal to the number of measurement sequences at a given test interval.

In yet another example of the implementation of the test method according to the invention, the measurement with a non-spectroscopic sensor embedded in the head 106 of probe 1 is carried out more than once in each test interval for averaging the result of a given measurement in the test interval or for rejecting, by the program that controls and analyzes the measurement results, results that are erroneous or significantly different from the values of the other measurements in the test interval.

In a further example of the implementation of the test method, based on the first example, before the first delivery of the unmanned aerial vehicle 2 of the system to the place where the measurement is performed, calibration (calibration) is carried out by performing a measurement with the spectroscope 120 of the probe 1 of the standard for input into the software that processes the results of the measurement of the reference value on the basis of the obtained reading for the standard. The reference measurement including calibration is carried out according to the following steps: before taking a series of measurements in the cultivation field by the unmanned aerial vehicle 2 of the system according to the invention, equipped with the probe 1 according to the invention, a measurement of the standard is carried out, after which the result of this measurement is transmitted wirelessly or wired to the results processing unit (computer, database server) equipped with software for processing the results of spectroscopic measurements. Subsequently, a soil test is carried out according to any of the previous examples of implementation of the method according to the invention, whereby the software processing the results of the measurements generated by the spectrometer 120 or sensor assembly 109, moisture sensor 119, makes a correction of the spectroscopy result based on the reference result of the pattern test. After the probe 1 movement sequence, the measurement sequence and the probe 1 ground egress sequence (without or with simultaneous ground surveying during the probe 1 ground egress), the unmanned aerial vehicle 2 of the system moves to the next survey point on the surveyed acreage and all probe movement sequences and measurement sequences are repeated, excluding calibration, which is carried out before the first probe 1 movement sequence and measurement sequence at the first point of ground surveying on the acreage.

In further examples of implementation, calibration is carried out before the measurement sequence at a given measurement point. In still further examples of implementation, calibration is carried out after the measurement sequence at the measurement point of the surveyed area.

In further examples of the implementation of the method, calibration is carried out in a calibration module embedded within the body 201 of the unmanned aerial vehicle 2 of the system, with the calibration module containing a settling pocket for the material standard (so-called standard).

In a further example of implementation of the method, based on one of the previous examples, the result of a test with a measurement instrument other than the spectroscope of the 106 probe head 1 is used by the software processing the test results to correct the measurement values obtained by spectroscopy or for correcting or averaging the results obtained from the other sensors of the 106 probe head 1, whereby in this example of implementation of the method, cycles of movement of the probe 1 and measurements are carried out analogously to one of the previous examples, after which, after the probe 1 is brought out of the ground, the unmanned aerial vehicle 2 of the system moves to the next measurement point in the surveyed area.

In a further example of the implementation of the method, prior to carrying out the soil properties test, the value of the probe 1's depth into the ground is entered in the control software of the unmanned aerial vehicle 2 of the system, at which depth the measurement sequence is performed, with the specified measurement depth being less than the maximum depth that can be reached by the probe 1 embedded in the linear-rotary drive mechanism 207 of the unmanned aerial vehicle 2 of the system.

In a further example of the implementation of the method, similar to the previous example, more than one depth of probe 1's penetration into the ground is determined before the unmanned aerial vehicle 2 of the system carries out a sequence of probe 1's movements and associated measurement sequences, at which depths the unmanned aerial vehicle 2 of the system carries out the measurement sequence.

In another example of the method implementation, in the course of its implementation, the control software of the unmanned aerial vehicle 2 of the system measures the value of the deviation from the level for the unmanned aerial vehicle 2 of the system - using the instrumentation of the autopilot module, and makes adjustments to the advancement speed of the probe 1 by reducing, stopping or changing the direction of rotation of the first motor 213 during its penetration into the ground to offset the effect of the unmanned aerial vehicle 2 of the system's repulsion from the ground level.

In another example implementation of the method, during the sequence of insertion of probe 1 into the ground, the control software of the unmanned aerial vehicle 2 of the system, measures the value of the current drawn by the motors for determining the point at which blade 105 makes contact with the ground and starts screwing probe 1 into the ground. Determining the point at which probe 1 begins to screw into the ground is done by identifying the increase in the value of the current drawn by the motors. In addition, this measurement is used to determine the hardness of the soil being tested by the method according to the invention.

In still further examples of the implementation of the method, which is obvious to the expert, it is possible to freely combine individual sequences and their elements, for obtaining measurements at different depths, with different angular values of the rotation of probe 1, both during the sinking and moving of probe 1 out of the ground, while the sinking and moving of probe 1 can be interrupted for the duration of the measurement sequence, or the measurement sequence can be implemented during the movement of the probe (sinking or moving out).

In an even further example of the implementation of the method according to the invention, more than one unmanned aerial vehicle 2 of the system, each equipped with a probe 1 is used, with the unmanned aerial vehicles 2 of the system conducting soil properties testing in parallel and independently of each other. What is obvious to the specialist, the use of more than one unmanned aerial vehicle 2 of the system does not affect the essence of the invention and is a further advantageous development of the invention, allowing to reduce the duration of soil properties testing on a given acreage.

In an even further example of the implementation of the method according to the invention, based on the previous example, the use of more than one unmanned aerial vehicle 2 of the system is used to carry out a parallel test of soil properties on more than one acreage, for example (non-limitingly) two unmanned aerial vehicles 2 of the system, each coupled with a probe1 are used to carry out a parallel test on two different cultivation acres.

It is evident to the specialist that, with the increase in the number of unmanned aerial vehicles 2 of the system implementing the method according to the invention, the control and communication with the systems and the measurement instrumentation contained therein or within the probe head 106 1 is based on analogous principles of communication and control as in the case of the use of a single unmanned aerial vehicle 2 of the system 2, which are described in the essence of the disclosure as well as in earlier examples of the implementation of the invention.

In a further example of implementation, a survey of soil properties is carried out in various crop fields. A ground vehicle, preferably an automobile, carrying a mobile launch platform for unmanned aerial vehicles 2 of the system, travels on roads between crop fields. Once the vehicle is within range of the crop field on which the survey is to be conducted, the unmanned aerial vehicle 2 of the system assigned to conduct the survey takes off from the mobile platform, autonomously moves to the first survey point on the acreage in question and begins surveying the acreage in question. Then, after the survey, the unmanned aerial vehicle 2 of the system waits on the surveyed acreage until the ground vehicle with the mobile platform comes within its range again and then returns autonomously aboard the mobile platform.

In yet another example of implementing a survey of soil properties using more than one unmanned aerial vehicle 2 of the system on a single cultivation field, the unmanned aerial vehicles 2 of the system take-off from a ground vehicle containing a mobile launch platform and move around the cultivation field. Once the measurement points come into range, the assigned unmanned aerial vehicle 2 of the system, autonomously, takes off from the mobile platform, moves to the first measurement point and begins taking measurements on the acreage. After taking a certain number of measurements, the unmanned aerial vehicle 2 of the system remains on the given areal and waits until the ground vehicle is again within range to return to the mobile launch platform.

In an even further example of the implementation of the method, the unmanned aerial vehicle 2 of the system returning to the mobile launch platform undergoes an operation to recharge its battery before being delivered to a new area to be tested according to the invention.

In yet another example of how to implement the method, a mobile launch platform for unmanned aerial vehicles 2 of the system is embedded on an agricultural vehicle.

In an even further example of implementation, the results of a survey carried out using unmanned aerial vehicles 2 of the system, in the form of a georeferenced map, provide the basis for planning precision tillage operations using an autonomous fertilizer device.

What is obvious to the specialist, in the preceding examples of the implementation of the way, unmanned aerial vehicles 2 of the systemoperate in autonomous mode or operate on the basis of commands issued by the operator (user) of unmanned aerial vehicles 2 of the system.

Furthermore, as is obvious to the specialist, and resulting from the particular configuration of the probe head 106 of the probe 1 embedded in the linear-rotary drive mechanism 207 used by the unmanned aerial vehicle 2 of the system to implement the method according to the invention, the measurement results are processed in a computer unit located within the body 201 of the unmanned aerial vehicle 2 of the system or within the body 101 of the probe head 106 of the probe 1, after which they may be stored on an internal storage medium or transmitted in real time via a transmitter or communication cable located within the body 101 of the probe 1 or within the body 201 of the unmanned aerial vehicle 2 of the system to an external receiver, for data processing in an external computer (or cloud computing or computing server).

In addition, as is obvious to the specialist, the data acquired during the operation of the unmanned aerial vehicle 2 of the system, uploaded to an external receiver (computer, server, cloud) in combination with GPS and depth data for a given measurement, are used by external software to create georeferenced maps, including mapping the results in three dimensions (on the surface and deep in the ground) for planning precise agronomic treatments.

What is also obvious to the specialist, in all the cited and further examples of manner implementation, the cycles of manner implementation are repeated by the unmanned aerial vehicle 2 of the system at all the previously indicated by the user land survey points in the given acreage starting from the first survey point, ending with the last survey point of the acreage.

In addition, in each of the cited examples of implementation of a method for investigating soil properties using an unmanned aerial vehicle 2 of the system coupled with a probe 1, the unmanned aerial vehicle 2 of the system may include software controlling its operation and allowing measurements to be made in autonomous mode, as well as the unmanned aerial vehicle 2 of the system and its functions may be controlled by the user through a computer program implemented on an external computer or via a suitable wireless remote control, which does not affect the essence of the disclosure and constitutes advantageous further possibilities of using the invention in realizing a method of conducting a soil properties test using the various elements disclosed in the application.

**List of designations**

| | | | |
|---|---|---|---|
| 1 - | probe | 2 - | unmanned aerial vehicle of the system |
| 101 - | body | | |
| 102 - | measuring part | 201 - | body |
| 103 - | settling part | 202 - | arm |
| 104 - | first end | 203 - | propeller |
| 105 - | blade | 204 - | leg |
| 106 - | head | 205 - | shock absorber |
| 107 - | inspection hole | 206 - | camera |
| 108 - | light source | 207 - | linear-rotary drive mechanism |
| 109 - | sensor assembly | 208 - | first handle |
| 110 - | electrode | 209 - | base |
| 111 - | battery | 210 - | second handle |
| 112 - | optical fiber | 211 - | cart |
| 113 - | bearing point | 212 - | guide |
| 114 - | partition | 213 - | first motor |
| 115 - | swivel joint | 214 - | screw |
| 116 - | guide | 215 - | second motor |
| 117 - | socket | 216 - | longitudinal serration |
| 118 - | mirror | 217 - | scraping ring |
| 119 - | moisture sensor | 218 - | rotary mechanism |
| 120 - | spectrometer | 219 - | drive belt |
| 121 - | entrance slit | 220 - | first pulley |
| 122 - | wireless communication module | 221 - | second pulley |
| | | 222 - | actuator |

## Claims

1. System for testing the soil properties, comprising unmanned aerial vehicle having a body provided with an electric power source, electronic steering arrangements and communication means capable of communicating with an external receiver and/or transmitter, GPS module, telemetric receiver and/or transmitter, wherein outside of body unmanned aerial vehicle has a plurality of radially protruding arms comprising motor-driven rotors, **characterized in that** a probe (1) is coupled by its drive mechanism with the body (201) of aerial vehicle, wherein the probe (1) has a tubular body (101) having a first conical end which contains a head (106), wherein the probe's (1) head has on the side of shell an inspection hole protected by glass, wherein on the outer surface of the head there is at least one helical blade (105) which is at least partially within screw line circumscribed around longitudinal axis of the probe's (1) body (101).

2. The system according to claim 1, **characterized in that** the probe's (1) blade (105) is located not higher than top surface of the unmanned aerial vehicle (2) body (201).

3. The system according to any of claim 1 or claim 2, **characterized in that** the probe's (1) drive mechanism, coupling probe (1) with body (201) of aerial vehicle (2) is a linear-rotary drive mechanism (207), preferably the linear drive and rotating drive of the linear-rotary drive mechanism (207) are independent of each other, preferably drives of linear-rotary drive mechanism (207) are electric drives.

4. The system according to any of claim 1 to 3, **characterized in that** inside of the probe's (1) body (101) there is at least one wire running from the head (106) to the opposite to head (106) end of the body (101).

5. The system according to any of claims 1 to 4, **characterized in that** the components of the probe (1), carrying out the measurement of soil properties, are communicated with an external receiver and an external transmitter, preferably the probe (1) contains an internal data carrier.

6. The system according to any of claims 1 to 5, **characterized in that** the probe (1) has at least one element that changes the direction of light beam, preferably the probe's (1) head (106) contains at least one source of light (108), preferably the probe's (1) head (106) contains at least one spectrometer (120), more preferably the probe's (1) head (106) contains at least one of the following: moisture sensor (109), soil conductivity sensor, temperature sensor, and soil acidity (pH) sensor, wherein electrodes (110) of embedded sensor are located on the outer surface of the head (106) shell.

7. The system according to any of claims 1 to 6, **characterized in that** the unmanned aerial vehicle (2) contains a device for analysis of the signal transferred by wire from the probe's (1) head (106), preferably the unmanned aerial vehicle (2) contains device for analysis of the signal transferred wirelessly from the probe's (1) head (106), wherein preferably the device for analysis of the signal contains at least one spectrometer (120).

8. The system according to any of claims 1 to 7, **characterized in that** the unmanned aerial vehicle (2) contains at least one external light source, preferably the unmanned aerial vehicle (2) contains at least one camera (106), preferably the system contains a proximity sensor, preferably the system contains a motor's rotation counter.

9. The system according to any of claims 1 to 8, **characterized in that** the system has controlling software capable of autonomous control of the system.

10. The method for conducting a soil property test using a system for testing the soil properties, while the method comprises inserting a probe of the system into the soil, with the longitudinal axis of the probe being perpendicular to the soil surface during the measurement, performing a soil property test using the components of the system's probe carrying out the measurement of soil properties, followed by removal of the probe from the soil, **characterized in that the** method employs a soil property test system according to any of claims 1 to 20, wherein the soil property test probe (1) is delivered to the place where the measurement is carried out by means of an unmanned aerial vehicle (2) of the system, being coupled with the vehicle (2) by the probe's (1) drive means embedded on the vehicle (2), then it is introduced in a linear-rotary motion into the soil, marking out in the soil a cylindrical measuring section - extending from the surface of the soil to the place where the probe (1) reaches a predetermined maximum depth, after which the direction of the linear-rotary motion is changed and the probe (1) is led out of the soil, while along the length of the measuring section the properties of the soil are tested at least once, whereby the test is carried out before leading the probe (1) out of the ground, by illuminating the soil surface tangent to the side of the body (101) of the probe (1) with a beam of light emitted by its source (108), collecting the light reflected from the soil surface tangent to the side of the body (101) of the probe (1) using the optical elements of the probe's head (106) and transmitting it in real time to the spectrum analysing device.

11. The method according to claim 10, **characterized in that** the testing of soil properties is carried out while inserting the probe (1) into the soil, preferably the testing of soil properties is carried out while the probe (1) is being brought out of the soil.

12. The method according to any of claims 10 to 11, **characterized in that** the linear-rotary motion of the probe (1) is periodically interrupted for the duration of the soil properties test, preferably the interruption of the linear-rotary motion of the probe (1) occurs at a predetermined depth of the soil.

13. The method according to any of claims 10 to 12, **characterized in that** the testing of soil properties further comprises at least one measurement of at least one of the following: electrical conductivity of the soil, moisture of the soil, temperature of the soil, and acidity of the soil, the testing being carried out using sensors built into the head (106) of the probe (1), preferably the testing of the soil properties includes carrying out at least one reference measurement, preferably the reference measurement includes at least one measurement of at least one of the following: electrical conductivity of the soil, moisture of the soil, temperature of the soil, and acidity of the soil, preferably the reference measurement includes calibration, preferably the reference measurement is used to calibrate the spectrum analysing device.

14. The method according to any of claims 10 to 13, **characterized in that** the measurement of the current drawn by the motor of the linear-rotary drive is used to measure the hardness of the soil, preferably the measurement of the current drawn by the motor of the linear-rotary drive is used to determine the moment of contact between the probe (1) and the ground, wherein preferably the measurement of the tilt value of the body (201) of an unmanned aerial vehicle (2) of the system is used to maintain the stability of the unmanned aerial vehicle (2) during the insertion of the probe (1) into the ground, wherein preferably the method includes measuring the depth of insertion of the probe (1) into the ground.

15. The method according to any of claims 10 to 14, **characterized in that** the method is carried out in parallel using more than one unmanned aerial vehicle (2) of a system, each coupled with a probe (1), preferably a parallel method of testing soil properties is carried out on more than one cultivation area at a given time.

16. The method according to any of claims 10 to 15, **characterized in that** at least one unmanned aerial vehicle (2) of the system takes off from the mobile launch platform before the start of the soil property measurement at the first measurement point on the respective acreage, and returns to the mobile launch platform after the completion of the soil property measurement at the last measurement point on the respective acreage.
